(19) Europäisches Patentamt
European Patent Office
Office européen des brevets

(11) **EP 3 202 438 B1**

(12) **EUROPEAN PATENT SPECIFICATION**

(45) Date of publication and mention
of the grant of the patent:
**23.10.2019 Bulletin 2019/43**

(21) Application number: **15847532.7**

(22) Date of filing: **16.09.2015**

(51) Int Cl.:
*A61M 1/18* (2006.01)    *A61M 1/14* (2006.01)
*A61M 1/36* (2006.01)    *A61M 1/16* (2006.01)

(86) International application number:
**PCT/JP2015/076321**

(87) International publication number:
**WO 2016/052204 (07.04.2016 Gazette 2016/14)**

(54) **ARTIFICIAL LUNG AND ARTIFICIAL HEART-LUNG CIRCUIT DEVICE**

KÜNSTLICHE LUNGE UND KÜNSTLICHE HERZ-LUNGEN-KREISLAUFVORRICHTUNG

POUMON ARTIFICIEL ET DISPOSITIF DE CIRCUIT COEUR-POUMON ARTIFICIEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2014 JP 2014201780**

(43) Date of publication of application:
**09.08.2017 Bulletin 2017/32**

(73) Proprietor: **Terumo Kabushiki Kaisha
Tokyo 151-0072 (JP)**

(72) Inventors:
• **SAITO, Takashi
Elkton, Maryland 21921 (US)**
• **SASAKI, Eisuke
Elkton, Maryland 21921 (US)**

(74) Representative: **Casalonga
Casalonga & Partners
Bayerstraße 71/73
80335 München (DE)**

(56) References cited:
WO-A1-2012/133372    JP-A- H06 237 992
JP-A- H06 237 992    JP-A- H06 237 993
JP-A- 2003 520 617    US-A- 5 706 889
US-A1- 2014 030 146

**Description**

Technical Field

The present invention relates to design and performance evaluation of an artificial lung.

Background of the invention

An artificial lung is an artificial organ which supplies oxygen to blood and removes carbon dioxide gas from blood, to replace or supplement the functions of a biological lung. Currently, an external circulation-type of artificial lung or oxygenator using a hollow fiber membrane is mainly adopted. Typically, the artificial lung can be used for up to several hours to support heart surgery during which the heart is stopped, wherein a so-called cardiotomy is used for forming an extracorporeal blood circulation circuit while the biological lung is typically not perfused. In addition, the artificial lung can also be utilized as a respiration assisting apparatus or a percutaneous cardiopulmonary assisting apparatus for a patient of acute pulmonary insufficiency or cardiac insufficiency.

In the artificial lung used in extracorporeal circulation of blood, since blood is guided out of the body of a patient, heat is taken from the blood. Therefore, when blood is returned to the patient, the blood is required to be rewarmed to be near the body temperature. In addition, a conventional extracorporeal circulation method sometimes employs a technique wherein the blood temperature is lowered so as to restrain metabolism of cells while the brain and central nerves are protected. Therefore, the artificial lung is normally provided with a heat exchanging element for adjusting a blood temperature.

The extracorporeal circulation circuit such as an artificial heart-lung circuit device is prepared for use by being filled with a priming solution or the like. A lactate Ringer solution or the like is used as the priming solution, and blood becomes diluted by a corresponding filling amount (blood filling amount) of the circuit. The diluted blood leads to a reduced effectiveness and a corresponding burden to a patient. In order to reduce the effects of diluted blood, a blood transfusion can be performed. However, the blood transfusion carries a risk of a physical burden or an infection occurring in a patient. Therefore, a reduction of the blood filling amount of the extracorporeal circulation circuit is desired. As a result thereof, in the artificial lung which is one of the components of the extracorporeal circulation circuits, reduction of the blood filling amount is desirable for the artificial lung as well.

In the design and performance evaluation of an artificial lung, it is favorable to have gas exchange performance of the artificial lung and heat exchange performance of the artificial lung to be as high as possible, and it is favorable to have the blood filling amount of the artificial lung and a blood side pressure loss of the artificial lung to be as low as possible.

For example, in a case where the blood filling amount is reduced so as to minimize a burden to a human body, improvements such as reducing the surface area of a gas exchanging hollow fiber membrane or the heat exchanging element and acquiring high performance through a small surface area by densely installing the gas exchanging hollow fiber membrane or the heat exchanging element may be required.

However, when the surface area of the gas exchanging hollow fiber membrane or the heat exchanging element is reduced, the gas exchange performance and the heat exchange performance thereof are deteriorated. When the gas exchanging hollow fiber membrane or the heat exchanging element are densely packed, there is concern of an increase of a blood side pressure loss and an increase of damage to the blood cells.

In the related prior art, each of the elements of the artificial lung has been improved independently from other elements (PTL 1), but there is a demand for a technology in which such various elements are united and the elements can be evaluated and examined regardless of the type and the size of the artificial lung.

Citation List

Patent Literature

[PTL 1] JP-T-2014-514963

Further relevant prior art is disclosed in documents US2014/030146 A1, JP H06 237992 A and US5706889 A.

Summary of the Invention

Technical Problem

There is provided a technology of designing and evaluating an artificial lung in which performance of each of elements of the artificial lung can be uniformly examined and a generally excellent performance can be exhibited, and there is provided an artificial lung exhibiting the generally excellent performance acquired by using this method.

Solution to Problem

The inventors have found that regardless of the difference of the type and the structure of the details of an artificial lung, when gas exchange performance, a heat exchange performance coefficient, a blood filling amount, and a blood side pressure loss are measured, performance factors of the artificial lung can be acquired through combinations of the measured results, and regardless of the type and the structure thereof, the artificial lung can be generally evaluated and compared through the acquired performance factors. Thus, the present invention has been realized.

An artificial lung according to present invention is defined independent claims 1 and 2.

In other words, below is provided according to the present invention.

(1) An artificial lung including a gas exchanger portion that is provided with an external circulation-type gas exchanging hollow fiber membrane bundle, and a wound heat exchanger portion that is provided with a heat exchanging element comprised of a resin tube. The artificial lung is designed such that when heat exchange performance coefficient of the artificial lung, a blood filling amount of the artificial lung, and a blood side pressure loss of the artificial lung are measured, and when $Fp3$=heat exchange performance coefficient of artificial lung/(blood filling amount of artificial lung×blood side pressure loss of artificial lung) is calculated, a value of $Fp3$ is equal to or greater than 0.15:

As used herein, heat exchange performance coefficient of the artificial lung is comprised of a dimensionless (i.e., scalar or absolute) number in accordance with conforming to ISO 7199 5.4.2,
blood filling amount of artificial lung is measured in the unit [mL] (1 mL=1 L/1,000) conforming to ISO 7199 5.3.3, and
blood side pressure loss of artificial lung is measured in the unit [mmHg/(L/min)].

(2) An artificial lung including a gas exchanger portion that is provided with an external circulation-type gas exchanging hollow fiber membrane bundle, and a wound heat exchanger portion that is provided with a heat exchanging element comprised of a resin tube. The artificial lung is designed such that when gas exchange performance of the artificial lung, a heat exchange performance coefficient of the artificial lung, a blood filling amount of the artificial lung, and a blood side pressure loss of the artificial lung are measured, and when $Fp$=(gas exchange performance of artificial lung×heat exchange performance coefficient of artificial lung)/(blood filling amount of artificial lung×blood side pressure loss of artificial lung) is calculated, a value of $Fp$ ranges from 1.5 to 2.5:
As used herein, the heat exchange performance coefficient of the artificial lung, the blood filling amount of the artificial lung, and the blood side pressure loss of the artificial lung are the same as those of (1) described above, Gas exchange performance of artificial lung can be derived according to a method and the unit [L/min] disclosed in Artificial Organ 16(1), 654-657 (1987), "Examination of Artificial Lung Performance Evaluation Method" by Nogawa, wherein the gas exchange performance is preferably determined in the unit [L/min]
(3) The artificial lung according to (1) or (2), wherein the artificial lung is designed such that when the heat exchange performance coefficient of the heat exchanger portion, the blood filling amount of the heat exchanger portion, and the blood side pressure loss of the heat exchanger portion are further measured, and when $Fp4$=heat exchange performance coefficient of heat exchanger portion/(blood filling amount of heat exchanger portion×blood side pressure loss of heat exchanger portion) is calculated, a value of $Fp4$ is equal to or greater than 1.5.
(4) The artificial lung according to any one of (1) to (3) . The gas exchange performance is a blood flow rate at which blood having oxygen saturation of 97.5% can be acquired in a case where the artificial lung is operated with hemoglobin concentration of 12.0 g/dL and oxygen saturation of 50% in venous blood.
(5) An artificial heart-lung circuit device including the artificial lung according to (1) to (4).

Advantageous Effect of Invention

In the artificial lung according to the present invention, regardless of the type and the difference of the structure of the details of the artificial lung, evaluation can be generally performed by using performance factors of $Fp$, $Fp3$, and $Fp4$, and thus, a generally excellent artificial lung performance can be acquired.

Brief Description of Drawings

Figs. 1A and 1B are cross-sectional views illustrating examples of structure of artificial lungs.
Fig. 2 is a schematic view describing an artificial heart-lung circuit device.

Detailed Description of Preferred Embodiments

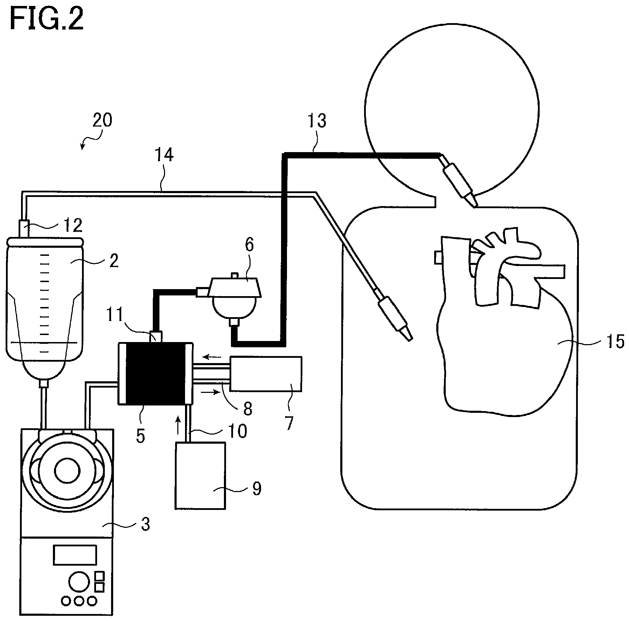

First, description will be given regarding an artificial heart-lung circuit device 20 of the present invention illustrated in Fig. 2. Fig. 2 is a conceptual circuit diagram of an example of an artificial heart-lung circuit device of the present invention and illustrates an artificial lung in which a heat exchanging element is internally mounted. A heat exchanger portion (not illustrated) that is provided with the heat exchanging element and a gas exchanger portion (not illustrated) that is provided with a hollow fiber membrane bundle are included within an artificial lung 5. The artificial heart-lung perfusion circuit 20 includes a venous blood reservoir 2 provided with a blood retention portion which retains blood and from which blood inside thereof can flow out by a blood supply pump 3. The artificial lung 5 is connected to a blood outlet port of the blood supply pump 3, and an artery filter 6 is provided on a downstream side of the artificial lung 5. A heat medium supplier 7 is provided which supplies a heat medium to the heat exchanger portion of the artificial lung 5, and a heat medium 8 supplied from the heat medium supplier 7 circulates in the artificial lung 5, thereby performing heat-exchanging. In addition, oxygen-containing gas 10 is supplied from an oxygen-containing gas supplier 9 to the inside of a hollow fiber membrane of the artificial lung 5.

Specifically, the artificial heart-lung perfusion circuit 20 interconnects the venous reservoir 2, a blood removal (venous blood) line 14 which is connected to a blood inlet port 12 of the vein reservoir 2 and through which blood removed from a heart 15 is received, and a blood supply (arterial blood) line 13 through which blood is supplied to the heart 15 through a blood outlet port 11 of the artificial lung 5 via the artery filter 6.

<Performance Factor of Artificial Lung in Entirety>

According to the present invention, in evaluation of an artificial lung, it is favorable to consider various factors, such as, gas exchange performance of the artificial lung [measured in L/min] and a heat exchange performance coefficient of artificial lung [measured as an absolute (i.e., dimensionless or scalar) number], which are preferably made as large as possible. Furthermore, it is desirable to have a blood filling amount of artificial lung [measured in mL] (1 mL=1 L/1, 000) and a blood side pressure loss of artificial lung [measured in mmHg/ (L/min)] to be as small as possible. In order to optimize performance of an artificial lung, the present invention defines a performance factor Fp of the gas exchanger portion and the heat exchanger portion of the artificial lung through the following Expression.

```
Fp=(gas   exchange   performance   of   artificial   lung
[L/min]×heat exchange performance coefficient of artificial
lung [absolute number])/(blood filling amount of artificial
lung  [L]×blood  side  pressure  loss  of  artificial  lung
[mmHg/(L/min)])
```

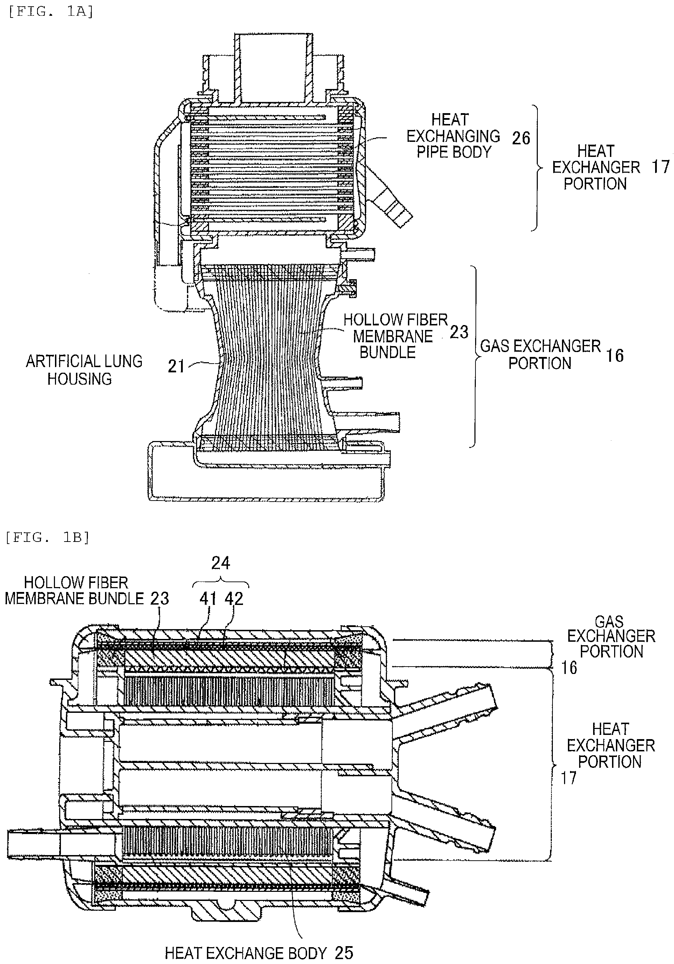

Fig. 1 illustrates cross-sectional views of two examples of external circulation-type artificial lungs in which hollow fiber membranes are used. In the artificial lung illustrated in Fig. 1 [Fig. 1A], a tubular body-shaped artificial lung housing 21 is formed and a hollow fiber membrane bundle 23 is internally accommodated, thereby configuring a gas exchanger portion 16. Above the gas exchanger portion 16, there is provided a heat exchanger portion 17 in which multiple heat exchanging pipe bodies 26 serving as the heat exchanging elements are disposed and blood flows in the heat exchanging pipe bodies 26 and is subjected to heat exchange.

In the artificial lung illustrated in Fig. 1 [Fig. 1B], a housing in its entirety has a cylindrical shape. A filter member 41 and an exhaust hollow fiber membrane layer 42 configure air bubble removal means 24 which functions as an artery filter are internally provided in a concentric cylindrical manner from a side close to the side wall of the housing, and the hollow fiber membrane bundle 23 is internally disposed in a concentric circle manner, thereby configuring the gas exchanger portion 16. A heat exchange body 25 serving a heat exchanging element is disposed further inside, thereby configuring the heat exchanger portion 17.

As described in the examples of Fig. 1, the artificial lung of the present invention is provided with at least the gas exchanger portion 16 and the heat exchanger portion 17. Examples of the gas exchanger portion include a region which is configured to have the gas exchanging hollow fiber membrane bundle 23 disposed in the artificial lung 5, and an external circulation blood portion surrounding the hollow fiber membrane bundle 23 and allowing blood to flow therein, and in which the hollow fiber membrane bundle 23 and the blood come into contact with each other. The heat exchanger portion 17 is configured to have the heat exchanging element in which blood flows, and a heat medium circulation chamber surrounding the heat exchanging element.

Description will be given regarding a method of measuring each of the factors for calculating a performance factor for optimizing performance of the artificial lung as a whole. 1) Gas exchange performance of the artificial lung can be measured in the units of [L/min] according to the method described in Artificial Organ 16(1), 654-657 (1987), "Examination of Artificial Lung Performance Evaluation Method" by Nogawa.

Below are the contents included in "Examination of Artificial Lung Performance Evaluation Method".

1-a) An artificial lung is considered to be a black box. Examples of inputs to the artificial lung include Sv: the rate of oxygenated hemoglobin in venous blood, Hb: the hemoglobin concentration of test blood, and QB: the blood flow rate. As an output from the artificial lung, $S_AO2$: the rate of oxygenated hemoglobin of the blood supply line can be examined. When the output with respect to all of the inputs is measured, the gas exchange performance of the artificial lung can be evaluated. However, it has been empirically learned that the conditions are not complete independent variables and are correlated to each other. Therefore, the evaluation can be simplified by reducing the independent variables.

1-b) In consideration of the quantity of Hb×QB× (1-SvO2), the quantity is a quantity which can be referred to as the oxygen vacancy quantity (the quantity of oxygen which can be received) with respect to diffusion. When the quantities are equal to each other between different types of artificial lungs, it is assumed that the diffusion states in the artificial lungs are similar to each other (called a diffusion stratum similarity theory).

1-c) Since the above-referenced theory is broadened to a turbulence-type artificial lung, the performance is improved due to an increase of the flow rate and an increase of the Reynold's number. Therefore, the phenomenon is described based on a varying diffusion coefficient D. Since Hb and Re have a positive correlationship, a correction coefficient of Re is taken in both Hb and QB, thereby acquiring a grounded theoretical expression broadened to the turbulence-type artificial lung.

1-d) According to ISO 7199 5.4.1, (1-$S_A$O2)=97.5% and Hb=12 g/dL,
X-axis: (1-SvO2)×Hb×QB, and Y-axis: (1-SvO2) are established. Here, since (1-SvO2) is determined by the Y-axis, (1-SvO2) is taken from the X-axis. Furthermore, the index of QB is removed. As a result thereof, a curve having the X-axis: (1-SvO2) and the Y-axis: QB is acquired.

1-e) It is extremely difficult to perform an experiment for drawing a graph of 1-d). However, since experimental data of (1-$S_A$O2)=97.5% can be calculated through an experiment other than (1-$S_A$O2) =97.5% by using a variable transformation method, a graph (not illustrated) can be drawn. The variable transformation method used here is a method in which in a case where a value expressed by a complex function f(x) is unknown, a variable acquired through the experiment other than (1-$S_A$O2)=97.5% transformed to a variable of the experimental data of (1-$S_A$O2)=97.5% such that x (unknown variable) of the function f(x) does not change.

In other words, the gas exchange performance of the artificial lung is defined as a blood flow rate at which oxygen saturation of blood flowing out when blood having the hemoglobin concentration of 12.0 g/dL and the oxygen saturation of 50% flows in the artificial lung becomes 97.5%. As the gas exchange performance of the artificial lung increases, the value of the blood flow rate becomes greater. However, it is difficult to acquire the blood flow rate through an experiment. The reason is that in the calculation of the gas exchange performance, the oxygen saturation on the outlet side (artery side) is an input and the blood flow rate is an output. Meanwhile, in an experiment, the oxygen saturation on the outlet side (artery side) becomes an output and the blood flow rate becomes an input. Thus, by using the variable transformation method disclosed in Artificial Organ 16(1), 654-657 (1987), "Examination of Artificial Lung Performance Evaluation Method" by Nogawa, it is possible to acquire the blood flow rate from several pieces of experimental data, that is, the value of the gas exchange performance. Artificial Organ 16(1), 654-657 (1987), "Examination of Artificial Lung Performance Evaluation Method" by Nogawa can be referred to for more details of the variable transformation method.

A method of measuring gas exchange performance of the present invention will be specifically described. The artificial lung is embedded in an appropriate test circuit, and oxygen gas is supplied to the artificial lung from an oxygen gas supplier. Blood is caused to flow into the artificial lung under conditions of arbitrary oxygen saturation and an arbitrary blood flow rate on an inlet side (vein side), and blood at the entrance and the exit of the artificial lung is taken, thereby acquiring the oxygen saturation. It is desirable that the hemoglobin concentration is 12.0±1 g/dL. It is desirable to set the conditions of the oxygen saturation and the blood flow rate on the inlet side (vein side) such that the oxygen saturation on the outlet side (artery side) does not exceed 99%.

By using the acquired value and the variable transformation method disclosed in Artificial Organ 16(1), 654-657 (1987), "Examination of Artificial Lung Performance Evaluation Method" by Nogawa, a blood flow rate QB is calculated so as to acquire a result having the hemoglobin concentration Hb of 12.0 g/dL, the oxygen saturation SvO2 of 50% in venous blood, and the oxygen saturation $S_A$O2 of 97.5% in arterial blood. The blood flow rate QB thereof corresponds to the gas exchange performance.

Specifically, measurement under several conditions is performed in this experiment, and the measurement results are evaluated according to,

[Math 1]

$$S_AO2/S_vO2 = f\{(1-S_vO2) \times Hb^{(1+cRe)} \times QB^{(1-cRe-cQB)}\} \quad ... \quad \text{Expression (1)}$$

Using substitution for cRe and cQB, and wherein,

$$SAO2 = 0.975$$

(that is, 97.5%)

$$SvO2 = 0.500$$

(that is, 50%), and

$$Hb = 12.0$$

, QB can be obtained. The QB is the gas exchange performance of the present invention.

2) Heat exchange performance coefficient of the artificial lung can be measured as an absolute, scalar number using the method of ISO 7199 5.4.2

Thus, a heat exchange performance coefficient R is determined as follows. R=[(temperature Bout of blood at blood outlet port of artificial lung)-(temperature Bin of blood at blood inlet port of artificial lung)]/[(temperature Win of heat medium at heat medium inlet port of heat exchanger portion of artificial lung) - (temperature Bin of blood at blood inlet port of artificial lung)]

As a test solution for a blood flow path, blood which is taken from a cow and is subjected to heparinization is used. A test is performed under conditions in which the temperature Bin of blood at the blood inlet port of the artificial lung is 30±1°C and the temperature Win of the heat medium at the heat medium inlet port of the heat exchanger portion of the artificial lung is 40±1°C.

The blood flow rate is the maximum blood flow rate of the artificial lung. Water is used as the heat medium, and the measurement is performed under the condition in which the flow rate of the heat medium is 10 L/min.

In the calculation of the heat exchange performance coefficient of the artificial lung, the total sum of the performance of heat exchange in the gas exchanger portion and heat exchange in the heat exchanger portion is measured as the heat exchange performance coefficient. Accordingly, the measurement value can be acquired regardless of the type and the configuration of the artificial lung.

3) Blood filling amount of the artificial lung can be measured in units of [mL] using the method described in ISO 7199 5.3.3,

wherein the dry weight of the artificial lung before being filled is measured on a scale. Thereafter, the artificial lung is filled with a physiological salt solution. The weight of the artificial lung after being filled is measured on the scale again, and the filling amount is calculated through the following expression.

$$\text{Filling amount} = (\text{weight after being filled-dry weight})/(\text{proportion 1.006 of physiological salt solution})$$

4) Blood side pressure loss of the artificial lung can be measured using units of [mmHg/(L/min)]

The artificial lung to be measured is embedded in an appropriate circuit, and the pressures at the blood inlet port and the blood outlet port are measured, thereby calculating the pressure loss through the following expression

wherein the blood flow rate is measured at the maximum blood flow rate of the artificial lung to be measured.

$$\text{Pressure loss} = (\text{inlet port pressure} - \text{outlet port pressure}) / (\text{maximum blood flow rate})$$

The blood temperature is set to $37 \pm 1°C$ and the hematocrit value is set to $35 \pm 1\%$. The test solution is measured by using blood of a cow.

The measurement value can be acquired regardless of the type and the configuration of the artificial lung product.

When the performance factor Fp of commercially available artificial lungs currently on the market is calculated by using the performance factors defined above, the result of Table 1 below is acquired.

[Table 1]

|    | Product A | Product B | Product C | Product D |
|----|-----------|-----------|-----------|-----------|
| Fp | 1.0       | 1.0       | 0.5       | 1.2       |

Other artificial lungs as disclosed in Examples 1 to 3 are described in greater detail below. The gas exchange performance of the artificial lung [L/min], the heat exchange performance coefficient of artificial lung [absolute, scalar number], the blood filling amount of artificial lung [mL] (1 mL=1 L/1,000), and the blood side pressure loss of artificial lung [mmHg/(L/min)] are measured through the measurement method disclosed above and Fp is calculated, thereby acquiring the result of Table 2 below. The artificial lungs of Examples 1 to 3 use arterial filtering for internally mounted-type artificial lungs. However, in the method of calculating the performance factor of the present invention, regardless of the form of internally mounting or externally mounting the artery filter, the performance factor can be calculated, evaluated, and compared to each other. Therefore, the method can be applied to various types of artificial lungs. In other words, as described in the measurement method above, in a case where the artery filter is internally mounted, the gas exchange performance of the artificial lung, the heat exchange performance coefficient of the artificial lung, and the blood side pressure loss of the artificial lung are the results reflecting the internally mounted state. Regarding the blood filling amount of the artificial lung, the portion of the artery filter can be relatively ignored with respect to the total blood filling amount. Therefore, the measurement can be performed whether or not the artery filter is internally mounted.

[Table 2]

| Item | Unit | Example 1 | Example 2 | Example 3 |
|------|------|-----------|-----------|-----------|
| Gas exchange performance | L/min | 8.2 | 8.2 | 8.2 |
| Heat exchange performance coefficient | - | 0.58 | 0.57 | 0.59 |
| Blood side pressure loss | mmHg/(L /min) | 15.7 | 15.7 | 15.7 |
| Blood filling amount | mL | 150 | 160 | 180 |
| Performance factor Fp | - | 2.0 | 1.9 | 1.7 |

According to the result of Table 2, it is verified that the artificial lung having the performance factor of Fp of the present invention ranging from 1.5 to 2.5 and preferably ranging from 1.7 to 2.1 has a sufficiently high performance factor compared to the performance factor of conventional artificial lungs available on the market, and when the performance factor of Fp is used during design and evaluation of an artificial lung, a generally excellent artificial lung can be designed, and the performance of the artificial lung can be appropriately evaluated.

2. Calculation of Fp3

<Simplified Performance Factor of the Artificial Lung in Its Entirety>

As an alternative performance factor for design and evaluation of an artificial lung device. In this embodiment, simplified measurement and calculation is based on defining a performance factor Fp3 having only the heat exchange performance coefficient of artificial lung [absolute number] as the numerator and blood filling amount of artificial lung [L] $\times$ blood side pressure loss of artificial lung [mmHg/ (L/min)] as the denominator.

$$Fp3=(\text{heat exchange performance coefficient of artificial lung [absolute number]})/(\text{blood filling amount of artificial lung [L]}\times\text{blood side pressure loss of artificial lung [mmHg/(L/min)]})$$

According to the result of Table 3, it can be understood that an artificial lung having Fp3 equal to or greater than 0.15 and preferably equal to or greater than 0.20 has a sufficiently high performance factor compared to the performance factor of the artificial lungs available on the market, and when the performance factor of Fp3 is used in design and evaluation of the artificial lung, a generally excellent artificial lung can be designed, and the performance of the artificial lung can be appropriately evaluated through the simplified measurement and calculation.

[Table 3]

| Item | Unit | Example 1 | Example 2 | Example 3 |
|---|---|---|---|---|
| Heat exchange performance coefficient | - | 0.58 | 0.57 | 0.59 |
| Blood side pressure loss | mmHg/( L/min) | 15.7 | 15.7 | 15.7 |
| Blood filling amount | mL | 150 | 160 | 180 |
| Performance factor Fp3 | - | 0.25 | 0.23 | 0.21 |

3. Calculation of Fp4

<Examination with Only Heat Exchanger Portion>

Regarding the artificial lungs of Examples 1 to 3 of the present invention, when an examination is performed while having only the heat exchanger portion as the subject, a performance factor Fp4 is examined while having heat exchange performance coefficient of the heat exchanger portion [absolute number] as the numerator and blood filling amount of heat exchanger portion [L] ×blood side pressure loss of heat exchanger portion [mmHg/(L/min)] as the denominator.

$$Fp4=(\text{heat exchange performance coefficient of heat exchanger portion [absolute number]})/(\text{blood filling amount of heat exchanger portion [L]}\times\text{blood side pressure loss of heat exchanger portion [mmHg/(L/min)]})$$

The blood filling amount of the heat exchanger portion indicated herein is calculated based on the volume of only a blood circulation chamber of the heat exchanger portion disposed in the artificial lung 5, and the blood side pressure loss of the heat exchanger portion is calculated by dividing the pressure difference between blood flowing into the heat exchanger portion and blood flowing out from the heat exchanger portion, by the maximum blood flow rate. According to Fp4, only the heat exchanger portion is taken as the subject, a heat exchanger portion excellent in the heat exchange performance can be designed, and the performance of the heat exchanger portion can be appropriately evaluated. Therefore, as a result, it is possible to design and evaluate an excellent artificial lung.
Description will be given regarding a method of measuring each of the factors for calculating the performance factor while focusing on only the heat exchanger portion of the artificial lung.
For the heat exchange performance coefficient of heat exchanger portion: the same measurement method and the same measurement values as those of the above-described heat exchange performance coefficient of the artificial lung in its entirety are used.
For the blood filling amount of artificial lung with only heat exchanger portion: since the unit [mL] and each of the dimensions of a heat exchange element of the heat exchanger portion are known, in a case where blood flows in

8

the heat exchange element, the volume thereof becomes the blood filling amount. In a case where the heat medium flows inside the heat exchange element and the surroundings thereof is filled with blood, the blood filling amount can be calculated through a method of estimating the filling amount based on a portion from which the volume of the heat exchange element is subtracted.

Blood side pressure loss of heat exchanger portion is determined based on the unit [mmHg/(L/min)]

The pressures at the blood inlet port and the blood outlet port of the heat exchanger portion are measured, and the pressure loss is calculated through the following expression, wherein the blood flow rate is measured at the maximum blood flow rate of the artificial lung product to be measured.

$$\text{Pressure loss=(inlet port pressure-outlet port pressure)/(maximum blood flow rate)}$$

The blood temperature is set to $37 \pm 1°C$ and the hematocrit value is set to $35 \pm 1\%$. The test solution is measured by using blood of a cow.

[Table 4]

| Item | Unit | Example 1 | Example 2 | Example 3 |
|---|---|---|---|---|
| Heat exchange performance coefficient | - | 0.58 | 0.57 | 0.59 |
| Blood side pressure loss | mmHg/(L /min) | 7.9 | 6.2 | 6.7 |
| Blood filling amount | mL | 35 | 60 | 70 |
| Performance factor Fp4 | - | 2.1 | 1.5 | 1.3 |

Meanwhile, when the performance factor Fp4 of the artificial lung with only the heat exchanger portion currently on the market is calculated by using the similar performance factor Fp4, the result of Table 5 below is acquired.

[Table 5]

| | Product A | Product B | Product C |
|---|---|---|---|
| Fp4 | 1.3 | 1.4 | 1.0 |

According to the results of Tables 4 and 5, when Fp4 exceeds 1.4, is preferably equal to or greater than 1.5, and is more preferably equal to or greater than 2.0, compared to the conventional products in the related art, it is possible to have a sufficiently high performance factor. When being combined with the gas exchanger portion having the gas exchange performance of the present invention, an artificial lung having an excellent performance factor can be obtained.

When the performance factor Fp4 of the present invention is used, the performance of the artificial lung with only the heat exchanger portion can be appropriately designed and evaluated as the subject. Therefore, as a result, it is possible to design and evaluate an artificial lung provided with the heat exchanger portion having excellent heat exchange performance.

[Examples]

The present invention will be specifically described below by using examples configured in a manner that satisfies the performance factors in the manner indicated. However, the present invention is not limited to the artificial lung and the manufacturing method of these examples.

(Examples 1 to 3)

In Examples 1 to 3, in order to design an artificial lung having a performance factor Fp more excellent than the performance factor Fp of the artificial lung currently on the market, shown in Table 1, the below-described improvement of the gas exchanger portion and the heat exchanger portion is designed and specified.

A gas exchanging hollow fiber is reduced in diameter, and the disposition of the hollow fiber membrane bundle is examined. In addition, a heat exchanging resin tube is employed as the heat exchanging element, and the material thereof and the heat-transfer area thereof are adjusted, thereby examining the disposition of the heat exchanging resin tube. The details are disclosed in Table 6.

The blood filling amount of the artificial lung in its entirety is designed so to be preferably equal to or smaller than 200 mL and to be more preferably equal to or smaller than 190 mL. The blood filling amount with only the gas exchanger portion is designed so to be preferably equal to or smaller than 65 mL and to be more preferably equal to or smaller than 60 mL.

All of Examples 1 to 3 adopt artery filter internally mounted-type artificial lungs. In this case, the blood filling amount of a portion of the artery filter can be ignored with respect to the total blood filling amount. Therefore, the performance factors calculated in Examples 1 to 3 are not influenced by the artery filter.

As a result of the examinations, each of the elements with respect to blood becomes highly dense and an artificial lung having a low blood filling amount and high performance can be designed and evaluated.

[Table 6]

| Item | Example 1 | Example 2 | Example 3 |
|---|---|---|---|
| Hollow fiber bundle | Small diameter polypropylene hollow fiber bundle | Small diameter polypropylene hollow fiber bundle | Small diameter polypropylene hollow fiber bundle |
| Gas exchange area | 1.9 m$^2$ | 1.9 m$^2$ | 1.9 m$^2$ |
| Gas exchanger portion | Hollow fiber bundle winding | Hollow fiber bundle winding | Hollow fiber bundle winding |
| Heat exchange element | Resin tube | Resin tube | Resin tube |
| Heat-transfer area | 0.49 m$^2$ | 0.44 m$^2$ | 0.6 m$^2$ |
| Heat exchanger portion | winding | Bamboo blind winding | Bamboo blind winding |
| Presence of artery filter internally mounted | Present | Present | Present |

Industrial Applicability

An artificial lung of the present invention, regardless of the difference of the type and the structure of the details thereof, gas exchange performance, a heat exchange performance coefficient, a blood filling amount, and a blood side pressure loss are measured and performance factors of the artificial lung can be acquired through combinations of the measured results. The artificial lung can be generally evaluated and compared through the acquired performance factors. Therefore, it is possible to generally evaluate and compare the artificial lungs suitable for various types of methods and structure such as a pediatric artificial lung, an extracorporeal circulation circuit used during cardiotomy, a respiration assisting apparatus for a patient of acute pulmonary insufficiency or cardiac insufficiency, and a percutaneous cardiopulmonary assisting apparatus. Thus, it is possible to expect that the present invention is effectively used in designing and manufacturing an artificial lung which can exhibit excellent performance.

Reference Signs List

2    VEIN RESERVOIR
3    BLOOD SUPPLY PUMP
5    ARTIFICIAL LUNG
6    ARTERY FILTER
7    HEAT MEDIUM SUPPLIER
8    HEAT MEDIUM
9    OXYGEN-CONTAINING GAS SUPPLIER
10   OXYGEN-CONTAINING GAS
11   BLOOD OUTLET PORT
12   BLOOD INLET PORT
14   BLOOD REMOVAL (VENOUS BLOOD) LINE

15 HEART
16 GAS EXCHANGER PORTION
17 HEAT EXCHANGER PORTION
20 ARTIFICIAL HEART-LUNG CIRCUIT DEVICE
21 ARTIFICIAL LUNG HOUSING
23 HOLLOW FIBER MEMBRANE BUNDLE
24 AIR BUBBLE REMOVAL MEANS
25 HEAT EXCHANGE BODY
26 HEAT EXCHANGING PIPE BODY
41 FILTER MEMBER
42 EXHAUST HOLLOW FIBER MEMBRANE LAYER

**Claims**

1. An artificial lung (5) comprising:

   a gas exchanger portion (16) that is provided with an external circulation-type gas exchanging hollow fiber membrane bundle (23); and
   a wound heat exchanger portion (17) that is provided with a heat exchanging element comprised of a resin tube, wherein the artificial lung (5) is designed such that when a heat exchange performance coefficient of the artificial lung (5), a blood filling amount of the artificial lung (5), and a blood side pressure loss of the artificial lung (5) are measured, and when Fp3=heat exchange performance coefficient of artificial lung/(blood filling amount of artificial lung [L]×blood side pressure loss of artificial lung [mmHg/(L/min)]) is calculated, a value of Fp3 is equal to or greater than 0.15.

2. An artificial lung (5) comprising:

   a gas exchanger portion (16) that is provided with an external circulation-type gas exchanging hollow fiber membrane bundle (23); and
   a wound heat exchanger portion (17) that is provided with a heat exchanging element comprised of a resin tube, wherein the artificial lung (5) is designed such that when gas exchange performance of the artificial lung (5), a heat exchange performance coefficient of the artificial lung (5), a blood filling amount of the artificial lung (5), and a blood side pressure loss of the artificial lung are measured, and when Fp=(gas exchange performance of artificial lung [L/min]×heat exchange performance coefficient of artificial lung)/(blood filling amount of artificial lung [L]×blood side pressure loss of artificial lung [mmHg/(L/min)]) is calculated, a value of Fp ranges from 1.5 to 2.5.

3. The artificial lung (5) according to Claim 1 or 2, wherein the artificial lung (5) is designed such that when the heat exchange performance coefficient of the heat exchanger portion (17), the blood filling amount of the heat exchanger portion (17), and the blood side pressure loss of the heat exchanger portion (17) are further measured, and when Fp4=heat exchange performance coefficient of heat exchanger portion/(blood filling amount of heat exchanger portion [L]×blood side pressure loss of heat exchanger portion [mmHg/(L/min)]) is calculated, a value of Fp4 is equal to or greater than 1.5.

4. The artificial lung (5) according to Claim 2, wherein the gas exchange performance is a blood flow rate at which blood having oxygen saturation of 97.5% can be acquired in a case where the artificial lung (5) is operated with hemoglobin concentration of 12.0 g/dL and oxygen saturation of 50% in venous blood.

5. An artificial heart-lung circuit device (20) comprising: the artificial lung (5) according to any one of Claims 1 to 4.

**Patentansprüche**

1. Künstliche Lunge (5), umfassend:

einen Gasaustauscherabschnitt (16), der mit einem gasaustauschenden Hohlfasermembranbündel (23) vom externen Zirkulationstyp versehen ist; und

einen gewickelten Wärmetauscherabschnitt (17), der mit einem Wärmetauscherelement versehen ist, das aus einem Harzrohr besteht,

wobei die künstliche Lunge (5) derart ausgebildet ist, dass, wenn ein Wärmeaustauschleistungskoeffizient der künstlichen Lunge (5), eine Blutfüllmenge der künstlichen Lunge (5) und ein blutseitiger Druckverlust der künstlichen Lunge (5) gemessen werden, und wenn Fp3 = Wärmeaustauschleistungskoeffizient der künstlichen Lunge / (Blutfüllmenge der künstlichen Lunge [L] x blutseitiger Druckverlust der künstlichen Lunge [mmHg/(L/min)]) berechnet wird, ein Wert von Fp3 gleich oder größer als 0,15 ist.

2. Künstliche Lunge (5), umfassend:

einen Gasaustauscherabschnitt (16), der mit einem gasaustauschenden Hohlfasermembranbündel (23) vom externen Zirkulationstyp versehen ist; und

einen gewickelten Wärmetauscherabschnitt (17), der mit einem Wärmetauscherelement versehen ist, das aus einem Harzrohr besteht,

wobei die künstliche Lunge (5) derart ausgebildet ist, dass, wenn die Gasaustauschleistung der künstlichen Lunge (5), ein Wärmeaustauschleistungskoeffizient der künstlichen Lunge (5), eine Blutfüllmenge der künstlichen Lunge (5) und ein blutseitiger Druckverlust der künstlichen Lunge gemessen werden, und wenn Fp = (Gasaustauschleistung der künstlichen Lunge [L/min] x Wärmeaustauschleistungskoeffizient der künstlichen Lunge) / (Blutfüllmenge der künstlichen Lunge[L] x blutseitiger Druckverlust der künstlichen Lunge [mmHg/(L/min)]) berechnet wird, ein Wert von Fp in dem Bereich von 1,5 bis 2,5 liegt.

3. Künstliche Lunge (6) nach Anspruch 1 oder 2,
wobei die künstliche Lunge (5) derart ausgebildet ist, dass, wenn der Wärmeaustauschleistungskoeffizient des Wärmetauscherabschnitts (17), die Blutfüllmenge des Wärmetauscherabschnitts (17) und ein blutseitiger Druckverlust des Wärmetauscherabschnitts (17) ferner gemessen werden, und wenn Fp4 = Wärmeaustauschleistungskoeffizient des Wärmetauscherabschnitts / (Blutfüllmenge des Wärmetauscherabschnitts [L] x blutseitiger Druckverlust des Wärmetauscherabschnitts [mmHg/(L/min)]) berechnet wird, ein Wert von Fp4 gleich oder größer als 1,5 ist.

4. Künstliche Lunge (5) nach Anspruch 2, wobei die Gasaustauschleistung eine Blutflussrate ist, bei der Blut, das eine Sauerstoffsättigung von 97,5 % aufweist, in einem Fall erworben werden kann, in dem die künstliche Lunge (5) mit einer Hämoglobinkonzentration von 12,0 g/dL und einer Sauerstoffsättigung von 50 % im venösen Blut betrieben wird.

5. Künstliche Herz-Lungen-Kreislaufvorrichtung (20), umfassend:
die künstliche Lunge (5) nach einem der Ansprüche 1 bis 4.

**Revendications**

1. Poumon artificiel (5) comprenant :

- une section d'échange de gaz (16), équipée d'un faisceau (23) de membranes en fibres creuses pour échange de gaz, du type à circulation externe,
- et une section d'échange de chaleur (17) à enroulement, équipée d'un élément échangeur de chaleur constitué d'un tube en résine,

lequel poumon artificiel (5) est conçu de telle sorte que, lorsque l'on mesure le coefficient de performance d'échange de chaleur du poumon artificiel (5), le volume remplissable par du sang du poumon artificiel (5) et la chute de pression côté sang dans le poumon artificiel (5) et que l'on calcule la quantité suivante :

$$Fp3 = (\text{coefficient de performance d'échange de chaleur du poumon artificiel})/(\text{volume remplissable par du sang du poumon artificiel [L]} \times \text{chute de pression côté sang dans le poumon artificiel [mmHg/(L/min)]})$$

on obtient pour Fp3 une valeur supérieure ou égale à 0,15.

2. Poumon artificiel (5) comprenant :

- une section d'échange de gaz (16), équipée d'un faisceau (23) de membranes en fibres creuses pour échange de gaz, du type à circulation externe,
- et une section d'échange de chaleur (17) à enroulement, équipée d'un élément échangeur de chaleur constitué d'un tube en résine,

lequel poumon artificiel (5) est conçu de telle sorte que, lorsque l'on mesure la performance d'échange de gaz du poumon artificiel (5), le coefficient de performance d'échange de chaleur du poumon artificiel (5), le volume remplissable par du sang du poumon artificiel (5) et la chute de pression côté sang dans le poumon artificiel (5) et que l'on calcule la quantité suivante :

$$Fp = (\text{performance d'échange de gaz du poumon artificiel}\ [\text{L/min}] \times \text{coefficient de performance d'échange de chaleur du poumon artificiel})/(\text{volume remplissable par du sang du poumon artificiel}\ [\text{L}] \times \text{chute de pression côté sang dans le poumon artificiel}\ [\text{mmHg/(L/min)}])$$

on obtient pour Fp une valeur de 1,5 à 2,5.

3. Poumon artificiel (5) conforme à la revendication 1 ou 2, lequel poumon artificiel (5) est conçu de telle sorte que, lorsque l'on mesure le coefficient de performance d'échange de chaleur de la section d'échange de chaleur (17), le volume remplissable par du sang de la section d'échange de chaleur (17) et la chute de pression côté sang dans la section d'échange de chaleur (17) et que l'on calcule la quantité suivante :

$$Fp4 = (\text{coefficient de performance d'échange de chaleur de la section d'échange de chaleur})/(\text{volume remplissable par du sang de la section d'échange de chaleur}\ [\text{L}] \times \text{chute de pression côté sang dans la section d'échange de chaleur}\ [\text{mmHg/(L/min)}])$$

on obtient pour Fp4 une valeur supérieure ou égale à 1,5.

4. Poumon artificiel (5) conforme à la revendication 2, dans lequel la performance d'échange de gaz est le débit de sang avec lequel on peut obtenir du sang présentant un taux de saturation en oxygène de 97,5 % en faisant fonctionner le poumon artificiel (5) avec du sang veineux présentant une concentration d'hémoglobine de 12,0 g/dL et un taux de saturation en oxygène de 50 %.

5. Dispositif de circulation coeur-poumon artificiel (20) comprenant un poumon artificiel (5) conforme à l'une des revendications 1 à 4.

[FIG. 1A]

HEAT
EXCHANGING 26
PIPE BODY

HEAT
EXCHANGER 17
PORTION

HOLLOW FIBER
MEMBRANE 23
BUNDLE

GAS EXCHANGER 16
PORTION

ARTIFICIAL LUNG
HOUSING 21

[FIG. 1B]

HOLLOW FIBER
MEMBRANE BUNDLE 23

24
41 42

GAS
EXCHANGER
PORTION
16

HEAT
EXCHANGER
PORTION
17

HEAT EXCHANGE BODY 25

FIG.2

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014514963 T
- US 2014030146 A1
- JP H06237992 A
- US 5706889 A

**Non-patent literature cited in the description**

- **NOGAWA.** Examination of Artificial Lung Performance Evaluation Method. *Artificial Organ,* 1987, vol. 16 (1), 654-657